# EUROPEAN PATENT APPLICATION

(11) **EP 2 982 333 A1**
(43) Date of publication of application: **10.02.2016**
(21) Application number: 14880145.9
(22) Date of filing: 17.10.2014
(51) Int. Cl.: A61B 90/00, A61B 1/00, A61B 1/04, A61B 17/28

(54) **SURGICAL DEVICE**

(30) Priority: 23.01.2014 JP 2014010595
(71) Applicant: OLYMPUS CORPORATION, Shibuya-ku Tokyo 151-0072 (JP)
(72) Inventor: USHIJIMA, Takanori, Tokyo 151-0072 (JP); MATSUI, Toshihiro, Tokyo 151-0072 (JP)
(74) Representative: Gunzelmann, Rainer
(86) International application number: PCT/JP2014/077705
(87) International publication number: WO 2015/111260

(57) **Abstract**

Provided is a surgical device that can give an accurate feeling of depth even when the surgical device is inserted in a horizontal direction on left and right images. Therefore, a plurality of indicators 55 are provided in a shaft section 52 and an interval d between the respective indicators 55 adjacent to each other is set to an interval for always setting an interval D between the respective indicators 55 on the left and right respective images to be larger than a shift amount |XL-YR| in a left-right direction of the corresponding indicators 55 between the left and right images when an image of the shaft section 52 is picked up in a state in which the shaft section 52 is orthogonal to optical axes Ol and Or of left and right image pickup optical systems 171 and 17r within a predetermined error range.

## Description

### Technical Field

The present invention relates to a surgical device suitable for surgical treatment performed under a three-dimensional video obtained by picking up images of the same target from different visual points.

### Background Art

In various observation apparatuses such as a surgical microscope and an endoscope, a stereoscopic observation apparatus that picks up images of an object from different visual points using two image pickup optical systems on the left and right has been conventionally known. In the stereoscopic observation apparatus of this type, picked-up left and right object images are, for example, simultaneously or alternately displayed on a monitor. An observer or the like can stereoscopically observe the object images by observing, via special eyeglasses or the like, the object images displayed on the monitor.

Concerning a surgical device used in such a stereoscopic observation apparatus, various techniques for causing a surgeon or the like to accurately grasp a sense of distance in observing a three-dimensional video have been proposed. For example, Japanese Patent Application Laid-Open Publication No. 2008-136671 (Patent Literature 1) discloses a technique for, by providing, in a laser probe inserted into and removed from a probe channel of a binocular stereoscopic endoscope, a non-translucent covering member that covers a distal end portion circumferential surface of a light guide fiber and a translucent covering member that covers a portion contiguous to a distal end portion of the light guide fiber, clearly distinguishing the distal end portion covered by the non-translucent member from a body cavity wall and the like while suppressing a phenomenon, for example, in which only one existing laser probe is projected on a stereoscopic display as two laser probes.

Incidentally, for example, in laparoscopic surgery, since direct operation of a treatment instrument (a surgical device) is performed in a limited space, in general, a trocar for a scope and a trocar for the treatment instrument are disposed in different positions and the treatment instrument is obliquely inserted with respect to an observation axis of a laparoscope. In recent years, multiflexible forceps, bending forceps, and the like have been sometimes used in single-hole type laparoscopic surgery performed by inserting a scope and a treatment instrument into a trocar in one place, robot surgery, and the like. These treatment instruments can be operated from, in particular, a direction substantially perpendicular to the observation axis of the laparoscope. Further, in the treatment instrument of this type, a reduction in diameter is attained for low invasion.

However, for example, when the treatment instrument reduced in diameter is operated perpendicularly to the observation axis of the laparoscope and in a horizontal direction on left and right images, even with a laparoscope capable of performing three-dimensional observation, it is difficult to obtain position information in a depth direction of the treatment instrument. Such a phenomenon is the same, for example, in a case in which surgical devices such as a needle and a thread are operated.

The present invention has been devised in view of the above circumstances and it is an object of the present invention to provide a surgical device that can give an accurate feeling of depth even when the surgical device is inserted in a horizontal direction on left and right images.

### Disclosure of Invention

### Means for Solving the Problem

A surgical device according to an aspect of the present invention is a surgical device used under observation of a stereoscopic observation apparatus that three-dimensionally displays, on a display section, left and right images obtained by picking up images of a target object with left and right image pickup optical systems from different visual points. The surgical device includes: an elongated section disposed in an image pickup region by the left and right image pickup optical systems; and two or more indicators provided in the elongated section. An interval between the respective indicators adjacent to each other is set to an interval for always setting an interval between the respective indicators on the left and right respective images to be larger than a left-right parallax amount obtained by the left and right image pickup optical systems when the image pickup is performed in a state in which the elongated section is orthogonal to optical axes of the left and right image pickup optical systems within a predetermined error range.

### Brief Description of the Drawings

Fig. 1 is a schematic configuration diagram of a surgical endoscope system;
Fig. 2 is a sectional view schematically showing a main part of a distal end portion of an endoscope;
Fig. 3 is an explanatory diagram showing a state of laparoscopic surgery performed using the surgical endoscope system;
Fig. 4 is a perspective view showing a distal end side of an IT knife;
Fig. 5 is a perspective view showing a distal end side of gripping forceps;
Fig. 6(a) is an explanatory diagram of a left image obtained by picking up an image of the IT knife;
Fig. 6(b) is an explanatory diagram of a right image obtained by picking up an image of the IT knife;
Fig. 7(a) is an explanatory diagram of a left image obtained by picking up an image of the IT knife;
Fig. 7(b) is an explanatory diagram of a right image obtained by picking up an image of the IT knife;
Fig. 8 is a perspective view relating to a first modification and showing the distal end side of the IT knife;
Fig. 9 is a perspective view relating to a second modification and showing the distal end side of the IT knife;
Fig. 10 is a perspective view relating to a third modification and showing the distal end side of the IT knife;
Fig. 11 is a perspective view relating to a fourth modification and showing the distal end side of the IT knife; and
Fig. 12 is a perspective view relating to a fifth modification and showing the distal end side of the IT knife.

### Best Mode for Carrying Out the Invention

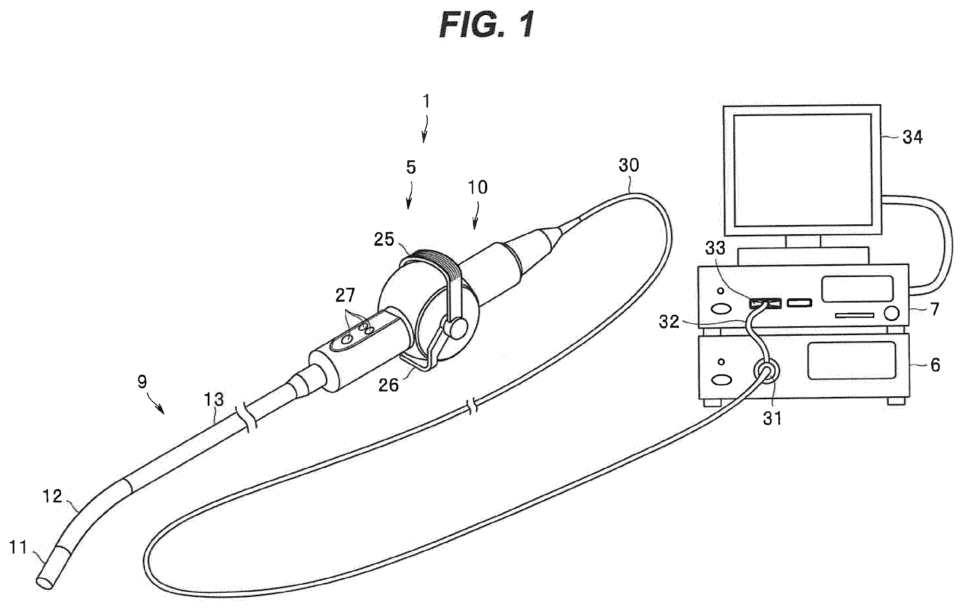

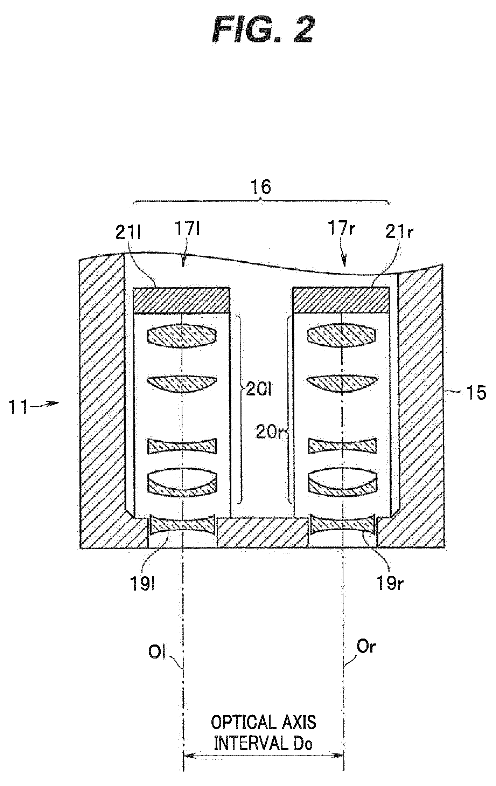

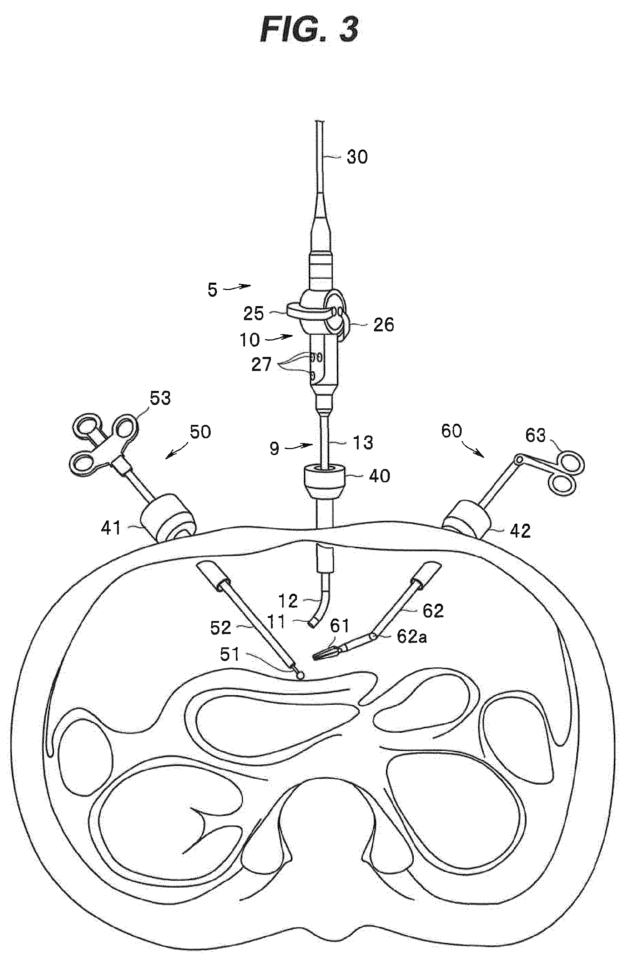

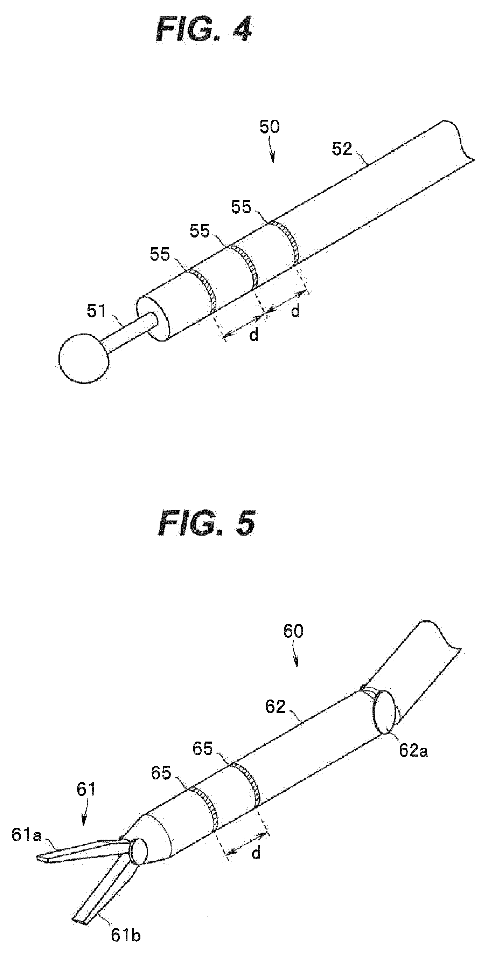

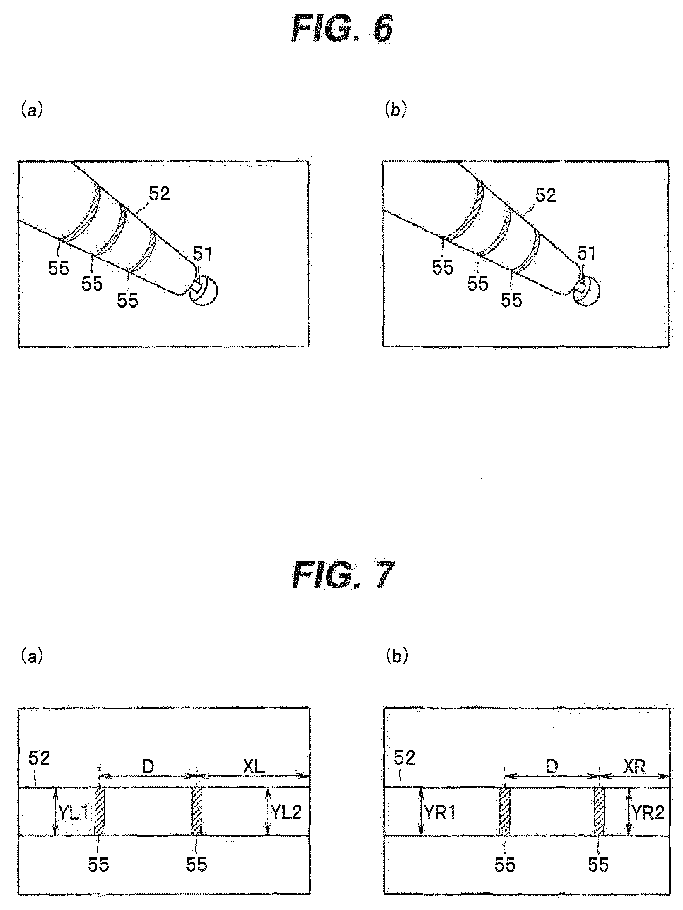

A mode of the present invention is described below with reference to the drawings. The drawings relate to an embodiment of the present invention. Fig. 1 is a schematic configuration diagram of a surgical endoscope system. Fig. 2 is a sectional view schematically showing a main part of a distal end portion of an endoscope. Fig. 3 is an explanatory diagram showing a state of laparoscopic surgery performed using the surgical endoscope system. Fig. 4 is a perspective view showing a distal end side of an IT knife. Fig. 5 is a perspective view showing a distal end side of gripping forceps. Fig. 6(a) is an explanatory diagram of a left image obtained by picking up an image of the IT knife. Fig. 6(b) is an explanatory diagram of a right image obtained by picking up an image of the IT knife. Fig. 7(a) is an explanatory diagram of a left image obtained by picking up an image of the IT knife. Fig. 7(b) is an explanatory diagram of a right image obtained by picking up an image of the IT knife.

A surgical endoscope system 1 shown in Fig. 1 includes an endoscope 5, which is an example of a stereoscopic observation apparatus, a light source apparatus 6, a camera control unit (CCU) 7, and a 3D monitor 34.

The endoscope 5 in the present embodiment is, for example, a rigid endoscope suitable for laparoscopic surgery. The endoscope 5 includes an insertion section 9 inserted into an abdominal cavity and an operation section 10 coupled to a proximal end portion of the insertion section 9.

The insertion section 9 is rigid and has length applied to the laparoscopic surgery. The insertion section 9 includes a distal-end rigid section 11, a bending section 12, and a rigid tube section 13 in order from a distal end side.

For example, as shown in Fig. 2, on an inside of a rigid-section main body 15, which is an armor member configuring the distal-end rigid section 11, a stereo optical system 16 for stereoscopically observing a subject or the like is disposed. More specifically, at a distal end of the rigid-section main body 15, objective lenses 191 and 19r for a left eye and a right eye are held in a state in which the objective lenses 191 and 19r have a predetermined optical axis interval Do to give a left-right parallax and are separated from each other. In the rigid-section main body 15, image-forming optical systems 201 and 20r for the left eye and the right eye formed by a plurality of lenses are respectively disposed on optical axes Ol and Or of the respective objective lenses 191 and 19r. Further, on a proximal end side in the rigid-section main body 15, image pickup devices 211 and 21r for the left eye and the right eye formed by solid-state image pickup devices (CCDs) or the like are disposed to be opposed to the left and right image-forming optical systems 201 and 20r. An image pickup optical system 171 for the left eye is configured by the objective lens 191 for the left eye, the image-forming optical system 201, and the image pickup device 211. An image pickup optical system 17r for the right eye is configured by the objective lens 19r for the right eye, the image-forming optical system 20r, and the image pickup device 21r. Further, the stereo optical system 16 is configured in the rigid-section main body 15 by the image pickup optical systems 171 and 17r for the left eye and the right eye.

As shown in Fig. 1, in the operation section 10, angle levers 25 and 26 for remotely operating the bending section 12 and various switches 27 for operating the light source apparatus 6, the CCU 7, and the like are provided. The angle levers 25 and 26 are, for example, levers capable of operating the bending section 12 in upward, downward, left, and right four directions.

A universal cord 30 is extended from a proximal end side of the operation section 10. A light guide connector 31 detachably connected to the light source apparatus 6 is provided at an extension end of the universal cord 30. Further, a proximal end side of a communication cable 32 inserted through the insertion section 9, the operation section 10, and the universal cord 30 branches from the light guide connector 31. A video connector 33 detachably connected to the CCU is provided at a proximal end of the communication cable 32.

The light source apparatus 6 supplies illumination light to an illumination optical system (not shown in the figure) provided in the distal-end rigid section 11. That is, a light guide (not shown in the figure) that optically connects the light guide connector 31 to the illumination optical system is inserted through the universal cord 30, the operation section 10, and the insertion section 9 of the endoscope 5. The light source apparatus 6 supplies the illumination light to the illumination optical system via the light guide.

Optical images of an object (an observation part, etc.) illuminated by the illumination light supplied from the light source apparatus 6 are picked up respectively by the left and right image pickup optical systems 171 and 17r. Image pickup signals generated by the left and right image pickup optical systems 171 and 17r are transmitted to the CCU 7 via the communication cable 32.

The CCU 7 includes, for example, an image-pickup-signal processing circuit that converts image pickup signals outputted by the image pickup devices 211 and 21r into predetermined video signals, a frame memory that stores, frame by frame, the respective video signals outputted by the image-pickup-signal processing circuit, and a 3D-video combination circuit that combines the video signals read out from the frame memory into a 3D video (all of which are not shown in the figure).

A video combined by the CCU 7 is outputted as a 3D video to the monitor 34 functioning as a display section capable of performing stereoscopic display of a polarization system. At this point, the object photographed at the optical axis interval Do between the optical axes Ol and Or of the left and right image pickup optical systems 171 and 17r and a predetermined distance to an observation target is projected on the monitor 34 with a left-right parallax amount. An observer such as a surgeon can fuse the object as an image having a cubic effect by viewing the monitor 34 with both the eyes via polarization glasses. Note that, as a system for realizing a stereoscopic view, besides displaying the image on the monitor 34 with the polarization system line by line, a method of displaying the image in a time division manner may be adopted. In laparoscopic surgery performed using such an endoscope system 1, for example, as shown in Fig. 3, a plurality of (in an example shown in the figure, three) trocars 40 to 42 are stabbed into an abdominal cavity wall. The insertion section 9 of the endoscope 5 is inserted through one trocar 40 among the trocars 40 to 42. A distal end side of the insertion section 9 of the endoscope 5 is inserted into an abdominal cavity according to the insertion through the trocar 40.

Various treatment instruments functioning as surgical devices are respectively inserted into the other trocars 41 and 42. Distal end sides of the various treatment instruments are inserted into the abdominal cavity according to the insertion through the trocars 41 and 42. In Fig. 3, as the treatment instruments, for example, an IT knife 50 and gripping forceps 60 are respectively inserted into the respective trocars 41 and 42.

For example, as shown in Figs. 3 and 4, the IT knife 50 includes a treatment section 51 including a high-frequency treatment electrode, a shaft section 52 functioning as an elongated section concatenated to the treatment section 51, and an operation section 53 concatenated to a proximal end side of the shaft section 52 and for remotely operating the treatment section 51.

For example, as shown in Figs. 3 and 5, the gripping forceps 60 include a treatment section 61 including a pair of forceps pieces 61a and 61b capable of opening and closing with respect to each other, a shaft section 62 functioning as an elongated section concatenated to the treatment section 61, and an operation section 63 concatenated to a proximal end side of the shaft section 62 and for remotely operating the treatment section 61. The gripping forceps 60 in the present embodiment include a joint section 62a halfway in the shaft section 62. The joint section 62a is capable of performing a bending action through operation applied to the operation section 63. That is, the gripping forceps 60 in the present embodiment are multiflexible forceps capable of causing a halfway part of the shaft section 62 to perform a bending action in the joint section 62a.

The shaft sections 52 and 62 of the IT knife 50 and the gripping forceps 60 are respectively formed in predetermined substantially equal thicknesses. Two or more indicators are provided on each of outer circumferential surfaces of the shaft sections 52 and 62.

More specifically, for example, as shown in Fig. 4, in the IT knife 50, three indicators 55 are disposed at every predetermined interval in a distal end side region of the shaft section 52. The indicators 55 are formed by, for example, linear patterns provided around the shaft section 52 in a right-angled direction to the shaft section 52 and formed in the same form one another.

For example, as shown in Fig. 5, in the gripping forceps 60, two indicators 65 are disposed a predetermined interval apart from each other in a distal end side region of the shaft section 62 (more specifically, for example, a region further on a distal end side than the joint section 62a). The indicators 65 are formed by, for example, annular patterns provided around the shaft section 62 in a right-angled direction to the shaft section 62 and formed in the same form each other.

In this case, intervals d among the respective indicators 55 provided on the shaft section 52 of the IT knife 50 and the interval d between the respective indicators 65 provided on the shaft section 62 of the gripping forceps 60 are set on the basis of, for example, a relation with left and right images picked up by the left and right image pickup optical systems 171 and 17r within an image pickup distance range set in advance.

More specifically, for example, as shown in Figs. 7(a) and 7(b), the interval d between the respective indicators 55 adjacent to each other on the shaft section 52 of the IT knife 50 is set to an interval for always setting an interval D between the respective indicators 55 on the left and right respective images to be larger than a parallax amount |XL-XR| in a left-right direction of the corresponding indicators 55 between left and right images observed via the monitor 34 when an image of the shaft section 52 is picked up in a state in which the shaft section 52 is orthogonal to the optical axes Ol and Or of the left and right image pickup optical systems 171 and 17r within a predetermined error range.

That is, the interval d between the respective indicators 55 adjacent to each other is set such that the interval D between the indicators on the left and right respective images always satisfies a relation of Expression (1) when an image of the shaft section 52 is picked up in a state in which the shaft section 52 is substantially orthogonal to the left and right optical axes Ol and Or. |XL-XR|<D (1)

In this case, the state in which the shaft section 52 is orthogonal to the optical axes Ol and Or of the left and right image pickup optical systems 171 and 17r within the predetermined error range means, for example, a case in which thicknesses close to both sides of the shaft section 52 projected on a left image (or on a right image) obtained by picking up an image of the shaft section 52 having an equal diameter coincide with each other within a predetermined error range (e.g., within an error range of 5%). For example, the state means a case in which, as shown in Fig. 7(a) (or Fig. 7(b)), when the shaft section 52 horizontally extends in a left-right direction on the left image (or the right image), thicknesses (heights) YL1 and YL2 (or YR1 and YR2) of the shaft section 52 close to both left and right sides on the images coincide with each other within the predetermined error range.

Similarly, the interval d between the indicators 65 adjacent to each other on the shaft section 62 of the gripping forceps 60 is also set to satisfy the relation described above.

By providing the indicators 55 and 65 in the shaft sections 52 and 62 of the IT knife 50 and the gripping forceps 60 in this way, it is possible to accurately give a feeling of depth using a three-dimensional image not only when the IT knife 50 and the like are inserted in a direction not orthogonal to the optical axes Ol and Or of the left and right image pickup optical systems 171 and 17r (see, for example, Figs. 6(a) and 6(b)) but also when the IT knife 50 and the like are inserted in a direction substantially orthogonal to the optical axes Ol and Or (see, for example, Figs. 7(a) and 7(b)).

That is, for example, as shown in Fig. 7, when the shaft section 52 and the like of the IT knife 50 are inserted in the horizontal direction on the left and right images and displayed traversing left and right both sides on the respective images in a state in which the shaft section 52 and the like are substantially orthogonal to the optical axes Ol and Or of the left and right image pickup optical systems 171 and 17r, it is sometimes difficult to grasp a difference between the left and right images due to a parallax only from images of the shaft section 52 and feel the feeling of depth. However, by providing a plurality of indicators 55 in the shaft section 52, it is possible to easily grasp the difference between the left and right images due to the parallax and feel the feeling of depth. In that case, the interval d between the respective indicators 55 adjacent to each other is set to an interval for always setting the interval D between the respective indicators 55 on the left and right respective images observed via the monitor 34 to be larger than a shift amount |XL-XR| in the left-right direction of the corresponding indicators 55 between the left and right images observed via the monitor 34 when an image of the shaft section 52 is picked up in the state in which the shaft section 52 is orthogonal to the optical axes Ol and Or of the left and right image pickup optical systems 171 and 17r within the predetermined error range. Consequently, it is possible to accurately prevent a deficiency in which, for example, different indicators 55 are displayed to overlap between the left and right images observed via the monitor 34. It is possible to accurately prevent, for example, misrecognition of the feeling of depth.

In this case, by providing the indicators 55 around an outer circumference of the shaft section 52, it is possible to accurately display the indicators 55 on images with respect to image pickup from all directions around the shaft section 52.

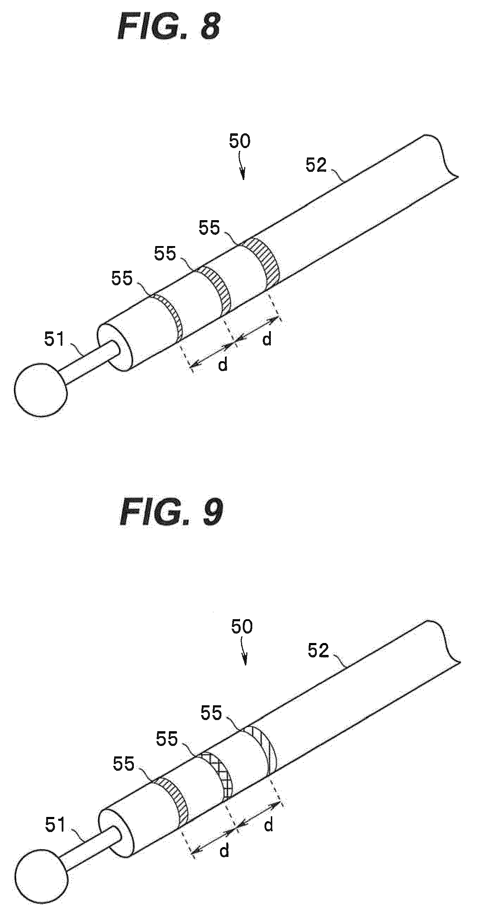

In order to more clearly distinguish the adjacent indicators 55 between the left and right images, for example, as shown in Fig. 8, it is also possible to set widths of the respective indicators 55 provided in the shaft section 52 to widths different from one another.

For example, as shown in Fig. 9, it is also possible to set colors of the respective indicators 55 provided in the shaft section 52 to colors different from one another.

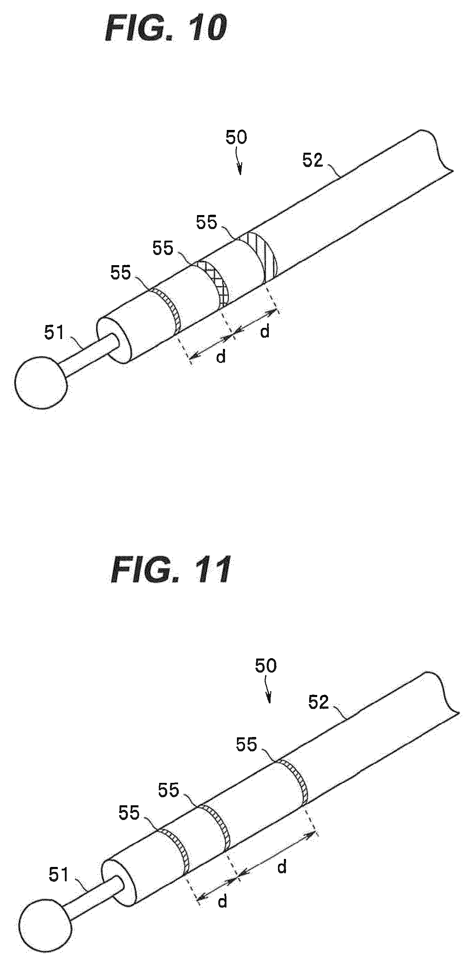

For example, as shown in Fig. 10, it is also possible to set the widths and the colors of the respective indicators 55 provided in the shaft section 52 to widths and colors different from one another.

For example, as shown in Fig. 11, it is also possible to set intervals among the respective indicators 55 provided in the shaft section 52 to intervals different from one another.

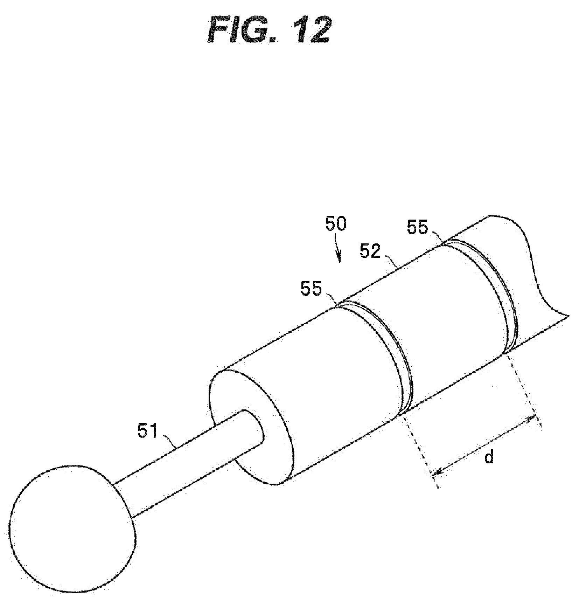

For example, as shown in Fig. 12, it is also possible to form the respective indicators 55 provided in the shaft section 52 with linear concave grooves instead of the linear patterns.

Although detailed description is omitted, naturally, it is possible to apply the same various modifications to the respective indicators 65 provided in the shaft section 62 of the gripping forceps 60.

It is possible to set forms of indicators of various surgical devices simultaneously used in the laparoscopic surgery or the like to be different from one another by, for example, adopting the indicators 55 shown in Fig. 12 and the indicators 65 shown in Fig. 5.

Note that the present invention is not limited to the respective embodiments described above. Various modifications and changes of the embodiments are possible. The modifications and the changes are also within a technical scope of the present invention. For example, naturally, the configuration described in the embodiment described above and the configurations described in the respective modifications may be combined as appropriate.

A surgical device applied with the present invention is not limited to the surgical device described above. Naturally, the surgical device can be applied to various treatment instruments including elongated sections such as shaft sections.

Further, the surgical device is not limited to treatment instruments. Naturally, the surgical device may be applied to needles, threads, and the like for surgical purposes operated using the treatment instruments.

A stereoscopic observation apparatus used together with the surgical device is not limited to the endoscope and may be naturally, for example, a microscope for brain surgery.

This application is based upon and claims the benefit of priority from Japanese Patent Application No. 2014-010595 filed in Japan on January 23, 2014, the contents of which are incorporated in the specification, the claims, and the drawings of this application.

## Claims

1. A surgical device used under observation of a stereoscopic observation apparatus that three-dimensionally displays, on a display section, left and right images obtained by picking up images of a target object with left and right image pickup optical systems from different visual points,
the surgical device comprising:
an elongated section disposed in an image pickup region by the left and right image pickup optical systems; and
two or more indicators provided in the elongated section, wherein
an interval between the respective indicators adjacent to each other is set to an interval for always setting an interval between the respective indicators on the left and right respective images to be larger than a left-right parallax amount obtained by the left and right image pickup optical systems when an image of the elongated section is picked up in a state in which the elongated section is orthogonal to optical axes of the left and right image pickup optical systems within a predetermined error range.

2. The surgical device according to claim 1, wherein the respective indicators are lines provided around an outer circumference of the elongated section.

3. The surgical device according to claim 1, wherein the respective indicators are lines provided around an outer circumference of the elongated section, and widths of the respective indicators are different from one another.

4. The surgical device according to claim 1, wherein the respective indicators are lines provided around an outer circumference of the elongated section, and colors of the respective indicators are different from one another.

5. The surgical device according to claim 1, wherein the respective indicators are lines provided around an outer circumference of the elongated section, and intervals among the respective indicators are different from one another.

6. The surgical device according to claim 1, wherein the respective indicators are linear concave grooves provided around an outer circumference of the elongated section.
